(19) Europäisches Patentamt
European Patent Office
Office européen des brevets

(11) Veröffentlichungsnummer: **0 646 378 A1**

## (12) EUROPÄISCHE PATENTANMELDUNG

(21) Anmeldenummer: **94114233.3**

(51) Int. Cl.6: **A61K 39/39**

(22) Anmeldetag: **09.09.94**

(30) Priorität: **30.09.93 DE 4333376**

(43) Veröffentlichungstag der Anmeldung:
**05.04.95 Patentblatt 95/14**

(84) Benannte Vertragsstaaten:
**CH DE DK FR GB IT LI SE**

(71) Anmelder: **GERBU Biotechnik GmbH**
**Am Kirchwald 6**
**D-69251 Gaiberg (DE)**

(72) Erfinder: **Grubhofer, Nikolaus, Dr.**
**Am Kirchwald 6**
**D-69251 Gaiberg (DE)**

(74) Vertreter: **Finsterwald, Manfred, Dipl.-Ing.,**
**Dipl.-Wirtsch.-Ing. et al**
**Robert-Koch-Strasse 1**
**D-80538 München (DE)**

(54) **Mittel zur Steigerung der Ausbeute von Antikörpern in der Immunologie.**

(57) Zur Erzielung einer verläßlicheren und intensiveren Immunantwort enthält das Mittel außer dem beschriebenen Glycopeptid eine Zinkverbindung und ein quaternäres Ammoniumsalz in synergistisch sich gegenseitig verstärkender Dosierung. Diese Kombination in Verbindung mit dem Antigen ist wesentlich leistungsfähiger als das früher beschriebene Mittel es ist einfach zu handhaben und wird vom Tier ohne erkennbare Beschwerden ertragen, so daß durch häufig wiederholte Applikation zusammen mit dem Antigen bisher noch nie gesehene Antigenausbeuten erzielt werden können.

Abbildung 1

Relativer Antikörpertiter nach 42 Tagen

A rel          VERSUCH NR.:

$A_{rel}$ = Antikörpertiter mit Adjuvantien im Verhältnis zu Antikörpertiter mit Freunds Adjuvans nach 28 Tagen. Zusammensetzung der Inkektion wie in Tabelle 1. Die dort mit * gekennzeichneten Versuchs- gruppen sind dargestellt.

EP 0 646 378 A1

Die Erfindung bezieht sich auf die Herstellung verbesserter Zubereitungen von immunologischen Adjuvantien, welche auf der Wirkung von Glycopeptiden basieren. Es war gezeigt worden, daß die Glycopeptide N-Acetylmuramyl-L-Alanyl-D-Isoglutamin (MDP) und N-Acetylglucosaminyl-N-Acetylmuramyl-L-Alanyl-D-Isoglutamin (GMDP) zusammen mit einem Antigen bei verschiedenen Wirbeltieren eine sehr gute Ausbeute an Antikörpern liefert, wenn man sie in der geeigneten Optimaldosierung in rein wäßriger Lösung anwendet. (DE 42 31 675.8)

Besonders an Kaninchen hat sich jedoch ergeben, daß bei manchen Tierpopulationen diese Wirkung nicht verläßlich eintritt, auch bestand generell der Wunsch nach verbesserten Antikörperausbeuten. Es wurde daher versucht, synergistisch wirkende Faktoren zu finden, welche die Wirkung der Glycopeptide steigern und stabilisieren können.

Auf der Suche nach solchen Synergisten diente als Richtschnur, daß die Glycopeptide aufgrund vorliegender wissenschaftlicher Befunde innerhalb 8 Stunden völlig aus dem Organismus verschwinden und während dieser kurzen Verweilzeit die Bildung von Interleukinen und makrophagen-modifizierende Polypeptiden auslösent, die ihrerseits nun die Vorgänge im Zuge der Immunantwort bestimmen. Dies alles kann nur unter Mitwirkung von Enzymen geschehen und daher wurde die Wirkung von Substanzen untersucht, welche als Coenzyme in Betracht kommen: Spurenelemente und Vitamine, weiterhin aber auch Verbindungen, welche an sich schon als Immunstimulatoren bekannt sind. Es wurde dabei mit einem Kaninchenstamm gearbeitet, welcher besonders schlecht auf GMDP als Einzelsubstanz anspricht.

Auch kam es darauf an, daß alle diese Substanzen ebenso körperfreundlich sind wie das GMDP selbst, so daß die Erhöhung der Wirksamkeit des Adjuvans nicht durch schlechtere Verträglichkeit erkauft werden muß.

Von den Spurenelementen wurden Kupfer, Mangan, Zink, Kobalt und Selen in die Studien einbezogen. Die weitaus deutlichste Wirkung zeigte sich bei Zink. Bei Kupfer und Selen war sie angedeutet, Mangan und Kobalt zeigen keine positive Wirkung.

Von den Vitaminen schien insbesondere das Vitamin E als immunogen prädestiniert, allerdings nur in wasserlöslicher Form, da Ölemulsionen vermieden werden sollten. Vitamin E phosphat und Vitamin E hemisuccinat gaben eine gewisse Erhöhung des Antikörpertiters. Ebenso Isoprinosin, eine als immunogen bekannte Mischung aus Inosin, Dimethylaminopropanol und p-Aminobenzoesäure.

Einige Detergenzien sind als Immunstimulatoren bekannt. So kamen Saponine, quaternäre Amine und zwitterionische Amine wie Cholamidopropyldimethylammoniopropansulfonsäure (CHAPS) in den Kreis der Untersuchung, ebenfalls Dextransulfat und Dextran. Von den quaternären Aminen zeigte eine beständige Wirksamkeit besonders das Dimethyldioctadecylmmoniumbromid (DDA).

Tabelle 1 fasst von den vielen Versuchen die hier relevanten zusammen. Die allerwichtigsten Ergebnisse kommen ausserdem noch in Tabelle 1 graphisch zur Dastellung.

Die eigentliche Überraschung im Zuge dieser Arbeiten bestand in der hervorstechenden Wirkung einer Kombination von GMDP mit Zink und Dimethyldioctadecylaminmmoniumbromid (DDA), welche weit über den additiven Effekt der Einzel komponenten hinausgeht.

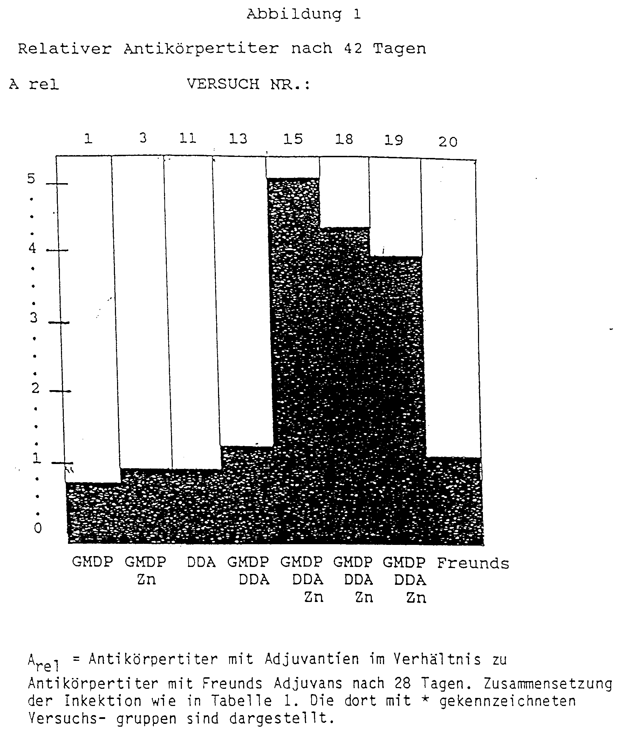

Die in Abbildung 1 wiedergegebenen Beispiele sowie zahlreiche weitere, hier nicht eigens festgehaltene Versuchskombinationen liefern Ausbeuten an Antikörpern, welche ganz allgemein im Bereich der Versuchsgruppe 14-18 lagen, teils noch erheblich darüber. Eine Adjuvansdosis von 10 $\mu$g GMDP + 100 $\mu$g Zink und 20 $\mu$g Dimethldioctylammoniumbromid (Versuchsgruppe 15) stellt in Bezug auf Effektivität und geringstmögliche Belastung der Tiere das zum gegenwärtigen Zeitpunkt erreichte Optimum dar, an welchem sich weitere Entwicklungsarbeiten zu orientieren haben werden. Auch die schon in DE 4231 675,8 präsentierte Optimalkonzentration des GMDP ließ sich bestätigen, unbeschadet der Zugabe verschiedener Synergisten.

Tabelle 1.

Verlauf des Antikörpertiters in Kaninchen mit verschiedenen Immunstimulatoren

| Nr | Immunstimulator | | | | $A_{rel}$ Tage nach Erstimpfung 28 | 42 | 56 |
|---|---|---|---|---|---|---|---|
| 1* | 10 µg GMDP | | | | 0.3 | 0.7 | 0.7 |
| 2 | 10 µg GMDP | 10 µg Zn | | | 0.5 | 0.9 | 1.1 |
| 3* | 10 µg GMDP | 100 µg Zn | | | 0.8 | 1.0 | 1.1 |
| 4 | 10 µg GMDP | 10 µg Cu | | | 0.4 | 0.9 | 1.0 |
| 5 | 10 µg GMDP | 100 µg Zn | 10 µg Cu | | 0.5 | 1.2 | 1.1 |
| 6 | 10 µg GMDP | 5 µg Se | | | 0.2 | 0.6 | 1.0 |
| 7 | 10 µg GMDP | 100 µg Tocopherylphosphat | | | 0.7 | 0.8 | 1.6 |
| 8 | 10 µg GMDP | 3 mg Isoprinosin | | | 0.9 | 0.9 | 0.8 |
| 9 | 10 µg GMDP | 100 µg Dextransulfat 40000 | | | 0.6 | 0.9 | 1.2 |
| 10 | 10 µg GMDP | 100 µg Dextran 40000 | | | 0.8 | 1.0 | 1.1 |
| 11* | 20 µg DDA | | | | 0.7 | 1.0 | 0.9 |
| 12 | 20 µg CHAPS | | | | 0.5 | 0.8 | 0.9 |
| 13* | 10 µg GMDP | 20 µg DDA | | | 1.0 | 1.3 | 1.2 |
| 14 | 10 µg GMDP | 20 µg CHAPS | | | 0.8 | 0.9 | 1.0 |
| 15* | 10 µg GMDP | 100 µg Zn | 20 µg DDA | | 1.3 | 5.2 | 4.3 |
| 16 | 10 µg MDP | 100 µg Zn | 20 µg DDA | | 1.1 | 2.9 | 3.1 |
| 17 | 10 µg GMDP | 250 µg Zn | 20 µg DDA | | 1.1 | 4.1 | 3.2 |
| 18* | 10 µg GMDP | 100 µg Zn | 100 µg DDA | | 1.0 | 4.4 | 5.2 |
| 19* | 10 µg GMDP | 250 µg Zn | 100 µg DDA | | 1.1 | 4.1 | 4.7 |
| 20* | Freunds vollständiges Adjuvans | | | | 1.00 | 0.9 | 0.8 |

$A_{rel}$ = Antikörpertiter mit Adjuvantien im Verhältnis zu Antikörpertiter mit Freunds Adjuvans nach 28 Tagen.

Versuchsbedingungen:

Tiere: Kaninchen: Inzuchtstamm b+Kap, schlechte GMDP-Responder.
Eine Versuchsgruppe bestand jeweils aus 4 Tieren
Antigen: Rinderserumalbumin 100 µg pro Tier
Injektion: Subcutan, 100 µl pro Injektion.
Zweite Injektion Antigen+Adjuvant nach 28 Tagen, bei Freunds mit FIA.
* Die so gekennzeichneten Vesuchsreihen sind in der Abbildung 1 graphisch dargestellt.

Abbildung 1                           ANLAGE
                                      ======

Relativer Antikörpertiter $A_{rel}$ nach 42 Tagen

| $A_{rel}$ Versuch Nr | 1 | 3 | 11 | 13 | 14 | 18 | 19 | 20 |
|---|---|---|---|---|---|---|---|---|
| | GMDP | GMDP Zn | DDA | GMDP DDA | GMDP DDA Zn | GMDP DDA Zn | GMDP DDA Zn | Freunds |

Für die praktische Darreichungsform erwies sich das schon in Anmeldung DE 42 31 675.8 angegebene L-Prolin als nützlich, indem es dem Lyophilisat Körper gibt und Leichtlöslichkeit des Dimethyldioctadecylammoniumbromides in der Antigenlösung vermittelt. Der in folgendem Beispiel beschriebene Zink-Prolinkomplex scheint in der Literatur nicht bekannt zu sein. Zinkgehalt 7.1 %, Verhältnis Zink/L-Prolin ca 1:7. Lässt sich aus Wasser, Alkohol und prolinhaltigen Lösungen in unveränderter Zusammensetzung umkristallisieren. Bei Reaktion von Zinkcarbonat- oder oxid mit Prolin in Ethanol erhält man viskose, anscheinend hochmolekulare Lösungen.

Beispiel 1:

Der Zink-Prolinkomplex wird hergestellt indem zu einer Suspension von 1.7 g Zinkhydroxidcarbonat in 50 ml kochendem Wasser 13.4 g L-Prolin portionenweise zugegeben werden. Wenig ungelöstes Material wird nach 10 Minuten abfiltriert, die Lösung im Vakuum eingedampft und im Exsikkator getrocknet. 14.4 g des Komplexes enthalten 1 g Zink.

Beispiel 2 :

2.9 g des trockenen Zink-Prolin-Komplexes werden in 50 ml Wasser gelöst dazu kommen 40 mg Dimethyldioctadecylammoniumbromid und 20 mg GMDP. Die Lösung wird durch ein 0.45 $\mu$m Filter feinfiltriert, je 100 $\mu$l davon in sterile Serumfläschchen dosiert und lyophilisiert. Ein Röhrchen enthält dann 1 mg Zn mit 13.4 mg Prolin in Komplexform, dazu 100 $\mu$g GMDP und 200 $\mu$g Dimethyldioctadecylammoniumbromid (DDA), ausreichend als Adjuvans für 10 Injektionen von Kaninchen.

**Patentansprüche**

1.  Mittel zur Steigerung der Ausbeute von Antikörpern in der Immunologie durch Injektion von Antigenen in Tiere, dadurch gekennzeichnet, daß sie Glycopeptide in Kombination mit Substanzen enthalten, welche die Wirksamkeit der Glycopeptide wesentlich steigern.

2.  Mittel nach Anspruch 1, dadurch gekennzeichnet, daß das Glycopeptid N-Acetylmuramyl-L-alanyl-D-isoglutamin (MDP) ist.

3.  Mittel nach Anspruch 1, dadurch gekennzeichnet, daß das Glycopeptid N-Acetylglucosaminyl-N-acetylmuramyl-L-alanyl-D-isoglutamin (GMDP) ist.

4.  Mittel nach Ansprüchen 1 - 3, dadurch gekennzeichnet, daß zweiwertige Übergangsmetallionen als Synergisten verwendet werden.

5.  Mittel nach Ansprüchen 1 - 4, dadurch gekennzeichnet, daß Zinkverbindungen als ergistische Substanz verwendet werden.

6.  Mittel nach Ansprüchen 1 - 5, dadurch gekennzeichnet, daß als synergistische Zinkverbindung ein Zink-Prolin-Komplex verwendet wird.

7.  Mittel nach Ansprüchen 1 - 6, dadurch gekennzeichnet, daß als synergistische Substanz ein quaternäres aliphatisches Amin verwendet wird.

8.  Mittel nach Ansprüchen 1 - 7 dadurch gekennzeichnet, daß das quaternäre Amin Dioctadecyldimethylammoniumbromid ist.

9.  Mittel nach Anspüchen 1 - 8 dadurch gekennzeichnet, daß die Synergisten Dextran oder Dextrnsulfat sind.

10. Mittel nach Ansprüchen 1 - 9 wobei die Synergisten in sich potenzierender Dosis miteinander kombiniert werden.

11. Mittel nach Ansprüchen 1 - 10 wobei eine Synergistenkombination von 1 Gew.Teil Glycopeptid, 10 Gew.Teil Zink in Form von 144 Teilen ZinkL-Prolinkomplex und 2 GewTeile Dimethyldioctadecylammoniumbromid zur Anwendung kommt.

Abbildung 1

Relativer Antikörpertiter nach 42 Tagen

A rel          VERSUCH NR.:

$A_{rel}$ = Antikörpertiter mit Adjuvantien im Verhältnis zu Antikörpertiter mit Freunds Adjuvans nach 28 Tagen. Zusammensetzung der Inkektion wie in Tabelle 1. Die dort mit * gekennzeichneten Versuchs- gruppen sind dargestellt.

| | **EINSCHLÄGIGE DOKUMENTE** | | EP 94114233.3 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 6) |
| D,P, A | <u>DE - A - 4 231 675</u> <br> (GERBU BIOTECHNIK GMBH) <br> * Ansprüche * <br> -- | 1-3 | A 61 K 39/39 |
| A | <u>EP - A - 0 315 153</u> <br> (SYNTEX (U.S.A.) INC.) <br> * Zusammenfassung; <br> Ansprüche * <br> -- | 1-3 | |
| A | <u>EP - A - 0 445 710</u> <br> (BEHRINGWERKE) <br> * Zusammenfassung * <br> -- | 1,4-6 | |
| A | <u>EP - A - 0 363 835</u> <br> (BEHRINGWERKE) <br> * Zusammenfassung * <br> -- | 1,4-6 | |
| A | <u>EP - A - 0 108 316</u> <br> (BAYER AG) <br> * Zusammenfassung * <br> ---- | 1,4-6 | |

RECHERCHIERTE
SACHGEBIETE (Int. Cl 6)

A 61 K

Der vorliegende Recherchenbericht wurde für alle Patentansprüche erstellt.

| Recherchenort | Abschlußdatum der Recherche | Prüfer |
|---|---|---|
| WIEN | 24-11-1994 | SCHNASS |